(19) Europäisches Patentamt
European Patent Office
Office européen des brevets

(11) **EP 2 329 809 A1**

(12) **EUROPEAN PATENT APPLICATION**
published in accordance with Art. 153(4) EPC

(43) Date of publication:
**08.06.2011 Bulletin 2011/23**

(21) Application number: **09808068.2**

(22) Date of filing: **19.08.2009**

(51) Int Cl.:
***A61K 8/41*** *(2006.01)* ***A61Q 5/10*** *(2006.01)*

(86) International application number:
**PCT/JP2009/003955**

(87) International publication number:
**WO 2010/021132 (25.02.2010 Gazette 2010/08)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **19.08.2008 JP 2008210269**

(71) Applicant: **Kao Corporation**
**Chuo-Ku**
**Tokyo 103-8210 (JP)**

(72) Inventors:
- **YAMAGUCHI, Masakazu**
  **Tokyo 131-8501 (JP)**
- **PRATT, Dominic**
  **64297 Darmstadt (DE)**

(74) Representative: **HOFFMANN EITLE**
**Patent- und Rechtsanwälte**
**Arabellastraße 4**
**81925 München (DE)**

(54) **HAIR DYE COMPOSITION**

(57) Provided is a hair dye composition utilizing a first agent containing an alkali agent and a second agent containing an oxidizing agent in mixture, the hair dye composition containing component (A) : a cationic anthraquinone direct dye represented by formula (1), and component (B): a compound represented by formula (2) or a salt thereof,
wherein R represents a hydrogen atom or an amino group; and $A^-$ represents an anion;
$R^1$ represents a hydrogen atom, a methyl group, or a hydroxyethyl group; $R^2$ represents a hydrogen atom, a methyl group, a phenyl group, a hydroxyethyl group, or a 3-(N-hydroxyethyl-N-(4-aminophenyl)aminc)-2-hydroxypropyl group; and $R^3$ represents a hydrogen atom, a hydroxyl group, a methyl group, a hydroxyethyl group or a hydroxyethoxy group.

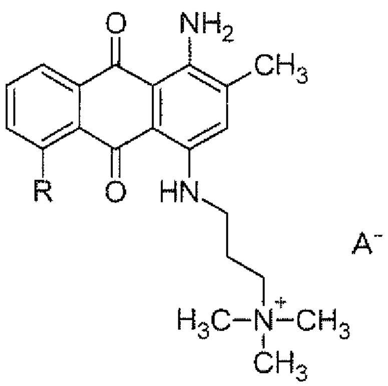
(1)

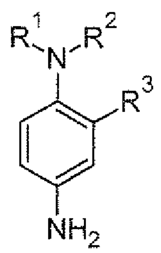
(2)

EP 2 329 809 A1

## Description

Field of the Invention

The present invention relates to a hair dye composition containing a cationic direct dye and an oxidation dye.

Background of the Invention

In hair dyes, various blue or violet direct dyes such as acid dyes, basic dyes and nitro dyes are used, in addition to the oxidation dyes that develop color as a result of a coupling reaction caused by an oxidizing agent, and thus adjustment of deep color tone has been widely carried out. However, in any case where these blue or violet direct dyes are used, such direct dyes are relatively easily lost from the hair due to hair washing or exposure to light. Therefore, the hair color immediately after hair dyeing is prone to color change, and this phenomenon is conspicuous in damaged hairs.

Thus, as one of the methods to solve the problems of the resistance (fastness) to light, hair washing, perspiration, friction and heat, there has been suggested a cationic anthraquinone direct dye having a trialkylammonium group (Patent Document 1). However, although this technical suggestion discloses that a number of cationic anthraquinone direct dyes can be equally used together with oxidation dyes, in order to actually produce a hair dye composition, shampoo fastness or the like of the hair color obtained by hair dyeing must be considered (see Non-Patent Document 1), and therefore, it is difficult to say that there has ever been suggested a cationic anthraquinone direct dye which is suitable for use in combination with oxidation dyes that those properties are taken into consideration.

Patent Document 1: EP-A1-1,820,826

Non-Patent Document 1: D.H. Johnson Ed., Hair and Hair Care, pp. 204-207, Marcel Dekker, New York, 1997

Summary of the Invention

The present invention provides a hair dye composition utilizing a first agent containing an alkali agent and a second agent containing an oxidizing agent in mixture, the composition including the following component (A) and component (B).

Component (A): Cationic anthraquinone direct dye represented by formula (1)

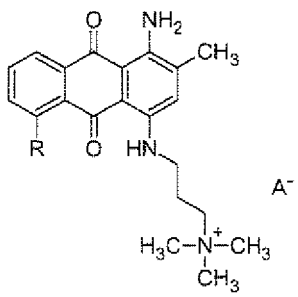

(1)

wherein R represents a hydrogen atom or an amino group; and $A^-$ represents an anion;

Component (B) : A compound represented by formula (2) or a salt thereof

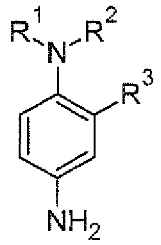

(2)

wherein $R^1$ represents a hydrogen atom, a methyl group or a hydroxyethyl group; $R^2$ represents a hydrogen atom, a methyl group, a phenyl group, a hydroxyethyl group, or a 3-(N-hydroxyethyl-N-(4-aminophenyl)amino)-2-hydroxypropyl group; and $R^3$ represents a hydrogen atom, a hydroxyl group, a methyl group, a hydroxyethyl group, or a hydroxyethoxy group.

The present invention also relates to a provision of a method of dyeing hair by applying the hair dye composition to the hair.

The present invention also relates to a provision of a method of dyeing hair by mixing the first agent and the second agent of the hair dye composition before use, subsequently applying the mixture to the hair, leaving the hair to stand, followed by washing away the hair dye composition, and drying the hair.

Detailed Description of the Invention

The present invention relates to a hair dye composition which has a high dyeing power, is capable of imparting a vivid color firmly to the hair, has excellent color fastness to light, washing, perspiration, friction and heat, and is less affected by decoloration with a lapse of time, and to a method of dyeing hair using the same.

The inventors of the present invention found that when a particular compound among the cationic anthraquinone direct dyes described in Patent Document 1 and a particular oxidation dye are used in combination in a hair dye composition, the problems described above can be solved.

The hair dye composition according to the present invention refers to a multi-part type hair dye composition such as a two-part type hair dye composition consisting of a first agent containing an alkali agent and a second agent containing an oxidizing agent, which are stored separately until immediately before use, or a three-part type hair dye composition consisting of these first agent and second agent as well as a third agent containing an oxidizing aid. The term "whole composition" means the entirety of the composition that has these agents in mixture and is actually applied to the hair. In addition, the term "two-part type" and the "three-part type" as used herein are intended to also include the aspects of using together a booster liquid that contains a cationic anthraquinone direct dye (A) and/or another direct dye, and such embodiments are similarly referred to as "two-part type" and "three-part type".

[Component (A)]

In the formula (1), examples of the anion $A^-$ include a chloride ion, a hydrogen sulfate ion, a methylsulfate ion, an acetate ion, and a 1/2 sulfate ion. The anion $A^-$ is preferably a methylsulfate ion. The cationic anthraquinone direct dye (A) is more specifically selected from the following compound (A-1) and compound (A-2), and (A-1) is more preferred.

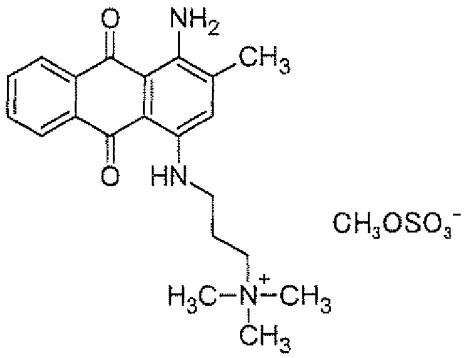

(A-1)

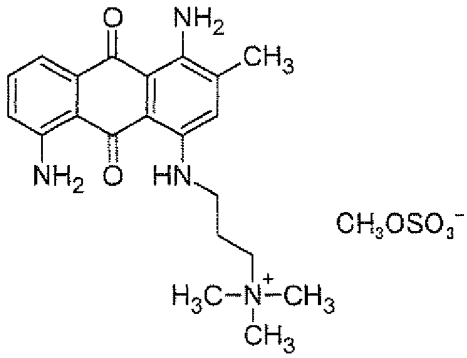

(A-2)

Any one of these compounds can be used alone, or the two can be used in combination. The content of the component (A) is preferably 0.01% to 5% by mass, more preferably 0.02% to 4% by mass, and even more preferably 0.05% to 3% by mass, relative to the entire composition.

[Component (B)]

The hair dye composition of the present invention includes a compound represented by the formula (2) or a salt thereof as an oxidation dye (B), together with the cationic anthraquinone direct dye (A). When these compounds are used together, highly vivid and highly colorfast dyeing with a deep color tone is made possible, which is not obtained by a direct dye alone or an oxidation dye alone. More specifically, the following oxidation dyes may be used.

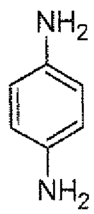

(B-1)

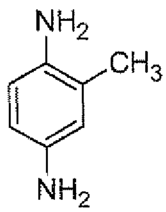

(B-2)

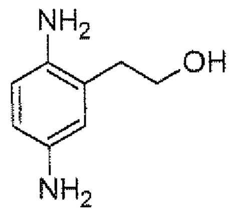

(B-3)

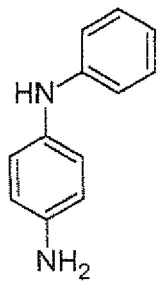

(B-4)

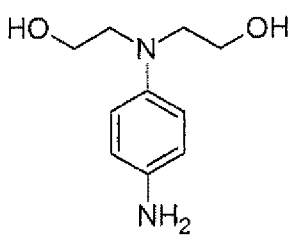

(B-5)

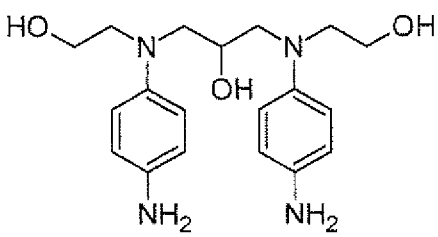

(B-6)

The oxidation dye (B) may be a free base or a salt, and preferred examples of the salt include hydrochloride, sulfate or acetate. Specifically, toluene-2,5-diamine (B-2) and paraphenylenediamine (B-1) are preferred. The oxidation dye (B) can be used singly, or two or more kinds can be used in combination. The content of the oxidation dye (B) is

preferably 0.1% to 5% by mass, and more preferably 0.5% to 3% by mass, relative to the whole composition.

[Other dye]

The hair dye composition of the present invention can have its color tone varied by incorporating a direct dye other than the component (A). As the direct dye other than the component (A), a known direct dye such as an acid dye, a basic dye, a nitro dye, a disperse dye or a cationic dye can be used. Examples of the direct dye include Blue No. 1, Violet No. 401, Black No. 401, Orange No. 205, Red No. 227, Red No. 106, Yellow No. 203, Yellow No. 403-(1). Acidic Orange 3, 2-nitro-p-phenylenediamine, 2-amino-6-chloro-4-nitrophenol, 3-nitro-p-hydroxyethylaminophenol, 4-nitro-o-phenylenediamine, 4-amino-3-nitrophenol, 4-hydroxypropylamino-3-nitrophenol, HC Blue 2, HC Orange 1, HC Red 1, HC Yellow 2, HC Yellow 4, HC Yellow 5, HC Red 3, N,N-bis(2-hydroxyethyl)-2-nitro-p-phenylenediamine, Disperse Violet 1, Disperse Blue 1, Disperse Black 9, Basic Blue 99, Basic Brown 16, Basic Brown 17, Basic Red 76, Basic Yellow 76, Basic Yellow 57, Basic Orange 31, and Basic Red 51.

Furthermore, for example, the direct dyes described in JP-A-2002-275040, JP-A-2003-107222, JP-A-2003-107223, JP-A-2003-113055, JP-A-2003-342139, JP-A-2004-107343, JP-A-2004-155746, JP-A-2004-262888 and JP-A-2006-182653 can also be added.

In the case of using another direct dye in combination, the total content of the cationic anthraquinone direct dye (A) and the other direct dye is preferably 0.05% to 20% by mass, more preferably 0.1% to 15% by mass, and even more preferably 0.1% to 10% by mass, relative to the whole composition.

The hair dye composition of the present invention may further contain an autoxidation dye represented by an indole or an indoline.

The total content of the cationic anthraquinone direct dye (A), the oxidation dye (B), the other direct dye and the autoxidation dye is preferably 0.2% to 25% by mass, more preferably 0.25% to 20% by mass, and even more preferably 0.5% to 10% by mass, relative to the whole composition.

[Component (C)]

The hair dye composition of the present invention may contain a surfactant (C). As the surfactant, any of a cationic surfactant, a nonionic surfactant, an amphoteric surfactant and an anionic surfactant can be used.

The cationic surfactant is preferably a mono-long chain alkyl quaternary ammonium salt, and specific examples thereof include cetyltrimethylammonium chloride, stearyltrimethylammonium chloride, arachyltrimethylammonium chloride, and behenyltrimethylammonium chloride.

Examples of the nonionic surfactant include polyoxyalkylene alkyl ether, polyoxyalkylene alkenyl ether, higher fatty acid sucrose ester, polyglycerin fatty acid ester, higher fatty acid mono- or diethanolamide, polyoxyethylene hardened castor oil, polyoxyethylene sorbitan fatty acid ester, polyoxyethylene sorbit fatty acid ester, alkyl saccharide surfactant, alkylamine oxide, and alkylamidoamine oxide.

Examples of the amphoteric surfactant include an imidazoline-based surfactant, a carbobetaine-based surfactant, an amidobetaine-based surfactant, a sulfobetaine-based surfactant, a hydroxysulfobetaine-based surfactant and an amidosulfobetaine-based surfactant.

Examples of the anionic surfactant include alkylbenzenesulfonate, alkyl or alkenyl ether sulfate, alkyl or alkenyl sulfate, olefin sulfonate, alkanesulfonate, saturated or unsaturated fatty acid salts, alkyl or alkenyl ether carboxylate, $\alpha$-sulfo fatty acid salts, N-acylamino acid type surfactants, phosphoric acidmono- or diester type surfactants, and sulfosuccinate. Examples of the alkyl ether sulfate include polyoxyethylene alkyl ether sulfate. Examples of the counterion for the anionic residues of these surfactants include an alkalimetal ion such as sodium ion or potassium ion; an alkaline earth metal ion such as calcium ion or magnesium ion; an ammonium ion; and an alkanolamine having 1 to 3 alkanol groups each having 2 or 3 carbon atoms (for example, monoethanolamine, diethanolamine, triethanolamine, or triisopropanolamine).

The surfactant can be used singly or in combination of two or more kinds, and there are no limitations on the content of the surfactant. However, the content is, for example, preferably 0.05% to 20% by mass, more preferably 0.1% to 18% by mass, and even more preferably 0.5% to 15% by mass, relative to the whole composition.

[Alkali agent]

The first agent of the hair dye composition of the present invention contains an alkali agent. Examples of the alkali agent include ammonia and salts thereof; an alkanolamine such as monoethanolamine, isopropanolamine, 2-amino-2-methylpropanol or 2-aminobutanol, and salts thereof; an alkanediamine such as 1, 3-propanediamine, and salts thereof; and a carbonate such as guanidine carbonate, sodium carbonate, potassium carbonate, sodium hydrogen carbonate or potassium hydrogen carbonate.

Among the alkali agents, ammonia, an alkanolamine and salts thereof are preferred. Preferred examples of the ammonium salt include ammonium carbonate and ammonium hydrogen carbonate, and preferred examples of the alkanolamine and salts thereof include monoethanolamine and salts thereof. Furthermore, it is most preferable that the content of these compounds fall in the range described below. The sum of the content (X) of ammonia and salts thereof calculated in terms of ammonia in the whole composition, and the content (Y) of monoethanolamine and salts thereof calculated in terms of monoethanolamine, is preferably 0.05% to 15% by mass, more preferably 0.1% to 10% by mass, and even more preferably 0.2% to 5% by mass, relative to the whole composition, from the viewpoint of obtaining sufficient effects of hair dyeing and bleaching, and from the viewpoint of reducing hair damage, scalp irritation and olfactory stimulation. Furthermore, the mass ratio of X/Y is preferably 0.01:1 to 2:1, more preferably 0.02:1 to 1:1, and even more preferably 0.05:1 to 0.5:1.

[Oxidizing agent]

The second agent of the hair dye composition of the present invention contains an oxidizing agent. The cationic anthraquinone direct dye (A) is stable against oxidizing agents. Thus, when the direct dye is mixed with the oxidizing agent and then the mixture is applied to the hair, dyeing and bleaching is simultaneously achieved, and a vivid dyeing color is obtained.

Examples of the oxidizing agent include hydrogen peroxide; a persulfate such as ammonium persulfate, potassium persulfate or sodium persulfate; a perborate such as sodium perborate; a percarbonate such as sodium percarbonate; and a bromate such as sodium bromate or potassium bromate. Among them, hydrogen peroxide is preferred from the viewpoint of bleachability for the hair, and the stability and effectiveness of the oxidizing agent itself. Furthermore, together with hydrogen peroxide, another oxidizing agent can be used in combination as an oxidizing aid. In this case, a composition containing an oxidizing aid can be used as a third agent, and thus a three-component hair dye composition can be obtained. The oxidizing aid is preferably a persulfate.

The oxidizing agent can be used singly, or two or more kinds can be used in combination, and the content of the oxidizing agent is preferably 0.5% to 30% by mass, and more preferably 1% to 20% by mass, relative to the whole composition. In the case of using hydrogen peroxide and a persulfate in combination, it is preferable that the content of hydrogen peroxide be 0.5% to 10% by mass of the whole composition, the content of the persulfate be 0.5% to 25% by mass of the entire composition, and the total content of the two compounds be 1% to 30% by mass.

The mixing ratio of the first agent containing an alkali agent, a cationic anthraquinone direct dye (A) and an oxidation dye (B), and the second agent containing an oxidizing agent in the hair dye composition of the present invention is preferably in the range of 2:1 to 1:3 as a volume ratio.

[Conditioning component]

The hair dye composition of the present invention may contain a conditioning component suitable for application to the hair. The conditioning component is a polymer or oil that can be dissolved or dispersed in the hair dye composition, and during rinsing or when diluted with water or shampoo, the conditioning component adheres to the hair.

When the conditioning component is used, the amount of incorporation thereof is preferably 0.01% to 30% by mass, more preferably 0.05% to 20% by mass, and even more preferably 0.1% to 10% by mass, relative to the whole composition.

Examples of the conditioning component suitable for the use in the hair dye composition of the present invention generally include cationic polymer, silicone, higher alcohol, and organic conditioning oil (for example, hydrocarbon oil, polyolefin and fatty acid ester).

Cationic polymers

A cationic polymer means a polymer having a cationic group or a group capable of being ionized into a cationic group, and in general, an amphoteric polymer acquiring cationicity is also included in the terminology. That is, the cationic polymer is a polymer containing an amino group or an ammonium group in a side chain of the polymer chain, or a polymer including a diallyl quaternary ammonium salt as a constituent unit, and examples thereof include cationized cellulose, cationic starch, cationic guar gum, a polymer or copolymer of a diallyl quaternary ammonium salt, and quaternized polyvinylpyrrolidone. Among these, from the viewpoint of softness, smoothness and easy finger-combing during shampooing, and easy manageability and moisture retention during drying, and from the viewpoint of stability of the agent, a polymer including a diallyl quaternary ammonium salt as a constituent unit, quaternized polyvinylpyrrolidone, and cationized cellulose are preferred, and a polymer or copolymer of a diallyl qua ternary ammonium salt, and cationized cellulose are more preferred.

Specific examples of the polymer or copolymer of a diallyl quaternary ammonium salt include dimethyldiallylammonium chloride polymer (polyquaternium-6, for example, MERQUAT 100; Nalco Company), dimethyldiallylammonium chloride/acrylic acid copolymer (polyquaternium-22, for example, MERQUAT 280, MERQUAT 295; Nalco Company), and dimethyldiallylammonium chloride/acrylic acid amide copolymer (polyquaternium-7, for example, MERQUAT 550; Nalco Company).

Specific examples of the quaternized polyvinylpyrrolidone include quaternary ammonium salts synthesized from a copolymer of vinylpyrrolidone (VP) and dimethylaminoethyl methacrylate, and diethyl sulfate (polyquaternium 11, for example, GAFQUAT 734, GAFQUAT 755 and GAFQUAT 755N (all by ISP Japan, Ltd.)).

Specific examples of the cationized cellulose include a polymer of a quaternary ammonium salt obtained by adding glycidyltrimethylammonium chloride to hydroxyethylcellulose (polyquaternium-10, for example, RHEOGUARD G and RHEOGUARD GP (all by Lion Corp.), POLYMER JR-125, POLYMER JR-400, POLYMER JR-30M, POLYMER LR-400 and POLYMER LR-30M (all by Amerchol Corp.)), and a hydroxyethylcellulose/dimethyldiallylammonium chloride copolymer (polyquaternium-4, for example, CELQUAT H-100, CELQUAT L-200 (all by National Starch and Chemical Company)).

The cationic polymer may be used in combination of two or more kinds. Furthermore, the cationic polymer gives better effects when the content is increased, but an excessively high content of the cationic polymer may cause stability failure and a decrease in the viscosity of the agent alone or during mixing. From this viewpoint, and from the viewpoint of enhancing the feel to the touch, the content of the cationic polymer is preferably 0.001% to 20% by mass, more preferably 0.01% to 10% by mass, and even more preferably 0.05% to 5% by mass, relative to the whole composition.

• Silicone

The hair dye composition of the present invention preferably contains silicone in order to have an excellent feeling of use imparted. Examples of the silicone include dimethylpolysiloxane, and modified silicone (for example, amino-modified silicone, fluorine-modified silicone, alcohol-modified silicone, polyether-modified silicone, epoxy-modified silicone, or alkyl-modified silicone), but dimethylpolysiloxane, polyether-modified silicone and amino-modified silicone are preferred.

The dimethylpolysiloxane may be any cyclic or non-cyclic dimethylsiloxane polymer, and examples thereof include SH200 series, BY22-019, BY22-020, BY11-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by Shin-Etsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF-6017, KF-6043, KF-353A, KF-354A, KF-355A (all by Shin-Etsu Chemical Co., Ltd.), FZ-2404, SS-2805, FZ-2411, FZ-2412, SH3771M, SH3772M, SH3773M, SH3775M, SH3749, SS-280X series, BY22-008 M, BY11-030, and BY25-337 (all by Dow Corning Toray Co., Ltd.).

The amino-modified silicone may be any silicone having an amino group or an ammonium group, and examples thereof include an amino-modified silicone oil having all or a part of the terminal hydroxyl groups capped with a methyl group or the like, and an amodimethicone which does not have the terminals capped. A preferred example of the amino-modified silicone may be a compound represented by the following formula (S).

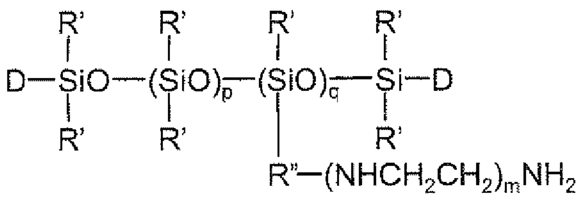

(S)

wherein R' represents a hydroxyl group, a hydrogen atom or $R^X$; $R^X$ represents a substituted or unsubstituted monovalent hydrocarbon group having 1 to 20 carbon atoms; D represents $R^X$, R"-$(NHCH_2CH_2)_mNH_2$, $OR^X$, or a hydroxyl group; R" represents a divalent hydrocarbon group having 1 to 8 carbon atoms; m represents a number from 0 to 3; p and q represent numbers, the sum of which is, as a number average, equal to or greater than 10 and less than 20,000, preferably equal to or greater than 20 and less than 3000, more preferably equal to or greater than 30 and less than 1000, and evenmore preferably equal to or greater than 40 and less than 800.

Specific examples of suitable commercially available products of the amino-modified silicone include amino-modified silicone oils such as SF8452C, SS-3551 (all by Dow Corning Toray Co., Ltd.), KF-8004, KF-867S, and KF-8015 (all by Shin-Etsu Chemical Co. , Ltd.); and amodimethicone emulsions such as SM8704C, SM8904, BY22-079, FZ-4671, and FZ-4672 (all by Dow corning Toray Co., Ltd.).

The total content of these silicones in the hair dye composition of the present invent ion is preferably 0.02% to 40% by mass, more preferably 0.1% to 20% by mass, and even more preferably 0.2% to 15% by mass, relative to the whole composition, from the viewpoint of obtaining sufficient effects and suppressing stickiness.

When the hair dye composition of the present invention contains a silicone and a cationic polymer, the mass ratio of the cationic polymer (active amount) :silicone in the whole composition is preferably 100:1 to 1:50, and more preferably 50:1 to 1:10.

• Higher alcohol

The hair dye composition of the present invention preferably contains, from the viewpoint of improving the sense of touch and stability, a higher alcohol in any one or more of the first agent, the second agent and the third agent. When the hair dye composition contains a higher alcohol, the higher alcohol forms a stricture with a surfactant to prevent separation of the hair dye composition, and also has an effect of improving the feel to the touch during rinsing.

The higher alcohol preferably has 8 to 22 carbon atoms, and more preferably 16 to 22 carbon atoms. Specific examples thereof include cetyl alcohol, stearyl alcohol, behenyl alcohol, and mixtures thereof.

The higher alcohol may be used in combination of two or more kinds, and the content is preferably 0.01% to 20% by mass, and more preferably 0.1% to 10% by mass, relative to the whole composition.

Organic conditioning oil

It is also preferable that the hair dye composition of the present invention contains an organic conditioning oil so as to have an excellent feeling of use imparted. The organic conditioning oil that is suitably used as a conditioning component is preferably a low-viscosity and water-insoluble liquid, and is selected from a hydrocarbon oil having at least 10 carbon atoms, a polyolefin, a fatty acid ester, a fatty acid amide, a polyalkylene glycol, and mixtures thereof. The viscosity of such an organic conditioning oil as measured at 40°C is preferably 1 to 200 mPa·s, more preferably 1 to 100 mPa·s, and even more preferably 2 to 50 mPa·s.

Examples of the hydrocarbon oil include a cyclic hydrocarbon, a linear aliphatic hydrocarbon (saturated or unsaturated), and a branched aliphatic hydrocarbon (saturated or unsaturated), and polymers or mixtures thereof are also included. The linear hydrocarbon oil preferably has 12 to 19 carbon atoms. The branched hydrocarbon oil includes hydrocarbon polymers, and preferably has more than 19 carbon atoms.

The polyolefin is a liquid polyolefin, more preferably a liquid poly-$\alpha$-olefin, and even more preferably a hydrogenated liquid poly-$\alpha$-olefin. The polyolefin used herein is prepared by polymerizing an olefin monomer having 4 to 14 carbon atoms, and preferably 6 to 12 carbon atoms.

The fatty acid ester may be, for example, a fatty acid ester having at least 10 carbon atoms. Examples of such a fatty acid ester include esters having a hydrocarbon chain derived from a fatty acid and an alcohol (for example, monoesters, polyhydricalcoholesters, ordi-and tricarboxylicacidesters). The hydrocarbon group of these fatty acid esters may have another compatible functional group such as an amide group or an alkoxy group as a substituent, or the hydrocarbon group may be covalently bonded to those functional groups. More specifically, an alkyl and alkenyl ester of a fatty acid having a fatty acid chain having 10 to 22 carbon atoms, a carboxylic acid ester of an aliphatic alcohol having an aliphatic chain derived from an alkyl and/or alkenyl alcohol having 10 to 22 carbon atoms, and a mixture thereof are suitably used. Specific examples of these preferred fatty acid esters include isopropyl isostearate, hexyl laurate, isohexyl laurate, isohexyl palmitate, isopropyl palmitate, decyl oleate, isodecyl oleate, hexadecyl stearate, decyl stearate, dihexadecyl adipate, lauryl lactate, myristyl lactate, cetyl lactate, oleyl stearate, oleyl oleate, oleyl myristate, lauryl acetate, cetyl propionate and dioleyl adipate.

Examples of the fatty acid amide include amides having a hydrocarbon chain derived from a fatty acid and an alkylamine or an alkanolamine. The hydrocarbon group of these fatty acid amides may have a substituent such as an amide group or an alkoxy group, and the hydrocarbon group may also be covalently bonded to the substituents. Specific examples of such a preferred fatty acid amide include oleic acid diethanolamide, lauric acid diethanolamide, coconut fatty acid amide, and coconut fatty acid diethanolamide.

Examples of the polyalkylene glycol include polyethylene glycol and polypropylene glycol, and a mixture of the two may be used, or a copolymer of ethylene oxide and propylene oxide may also be used.

The organic conditioning oil may be used in combination of two or more kinds, and the content thereof is preferably 0.1% to 25% by mass, and more preferably 0.5% to 20% by mass, relative to the whole composition.

[Medium]

The hair dye composition of the present invention uses water, and if necessary, an organic solvent, as a medium. Examples of the organic solvent include lower aliphatic alcohols such as ethanol, propanol and isopropanol; aromatic alcohols such as benzyl alcohol and benzyloxyethanol; polyols such as propylene glycol, 1,3-butanediol, diethylene glycol and glycerin; cellosolves such as ethylcellosolve and butylcellosolve; and carbitols such as ethylcarbitol and butylcarbitol.

There are no particular limitations on the content of the organic solvent, but the content is, for example, preferably 0.05% to 20% by mass, more preferably 0.1% to 15% by mass, and even more preferably 0.5% to 10% by mass, relative to the whole composition.

[Other components]

The hair dye composition of the present invention may contain, in addition to the components described above, other components that are conventionally used as cosmetic rawmaterials. Examples of these components include animal and plant oils and fats, higher fatty acids, natural or synthetic polymers, ethers, proteins, protein hydrolysates, amino acids, preservatives, chelating agents, stabilizers, oxidation inhibitors, plant extracts, herbal medicine extracts, ultraviolet absorbers, vitamins, dyes, and fragrances.

[pH]

The cationic anthraquinione direct dye (A) used in the hair dye composition of the present invention has excellent storage stability in a wide range of pH from 2 to 14, which is used with conventional hair dyes, and can be used in the pH range employed for the conventional oxidation hair dyes. However, from the viewpoint of the hair dyeing and bleaching effects of the hair dye composition and skin irritancy, the pH (25°C) of the composition as a whole during use (during mixing) is preferably 5 to 14, and more preferably 6 to 12. Furthermore, the pH of the first agent before mixing is preferably 7 to 14, and the pH of the second agent before mixing is preferably 2 to 5. As the pH adjusting agent, an inorganic acid such as hydrochloric acid or phosphoric acid; an organic acid such as citric acid, glycolic acid or lactic acid; a hydrochloride such as monoethanolamine hydrochloride; or a phosphate such as monopotassium dihydrogen phosphate or disodium monohydrogen phosphate, can be used in addition to the alkali agent.

[Formulation]

The hair dye composition of the present invention is preferably provided as a two-part type composition consisting of a first agent containing an alkali agent and a second agent containing an oxidizing agent such as hydrogen peroxide. The hair dye composition may also be a three-part type composition which further includes, in addition to the first agent and the second agent, a third agent containing an oxidizing aid.

In the case of the two-part type (or three-part type), the formulation of the first agent and the second agent may be made into, for example, liquid, emulsions, creams, gels, pastes, mousses or the like, and can also be prepared into an aerosol form. It is desirable for the hair dye composition of the present invention to obtain a viscosity that does not easily cause dripping when the first agent and the second agent (in the case of the three-part type, together with the third agent) are mixed and applied to the hair. It is preferable for the hair dye composition of the present invention, regardless of whether the composition is of the two-part type or the three-part type, to have the viscosity of the whole composition as measured at 25°C using a type B rotary viscometer equipped with a helical stand (B8R type viscometer; Tokimec, Inc.), in the range of 2000 to 100,000 mPa·s. Here, the viscosity is a value obtained by rotating the composition for one minute at 10 rpm using a Rotor T-C.

[Hair dyeing method]

In order to perform a hair dyeing treatment using the composition of the present invention, for example, the first agent and the second agent (in the case of the three-part type, together with the third agent) of the composition of the present invention may be mixed just before use, applied to the hair and left to stand for a predetermined time period of one hour or less, subsequently rinsed, and then dried. The temperature for application to the hair is preferably 15°C to 45°C, and the application time is preferably 3 to 50 minutes, more preferably 5 to 40 minutes, and even more preferably 10 to 30 minutes. In this case, when the hair dye is first lightly rinsed with water, and subsequently the hair is shampooed using a shampoo containing an anionic surfactant and then rinsed with water, the cationic polymer moderately flows out, while the silicone appropriately remains on the hair, exhibiting satisfactory conditioning effects. The shampoo is suitably an ordinary aqueous shampoo containing about 5% to 20% by mass of an anionic surfactant such as sodium

laureth-1 sulfate, sodium laureth-2 sulfate or sodium laureth-3 sulfate.

EXAMPLES

Examples 1 to 3

A first agent having the composition indicated in Table 1 and a second agent A having the composition indicated in Table 2 were prepared by a conventional method. The first agent and the second agent A thus obtained were mixed at a mass ratio of 1:1, and then dyeing properties was evaluated according to the following evaluation method of dyeing properties. The results are presented in Table 3.

[Evaluation method of dyeing properties]

The mixture of the first agent and the second agent A at a bath ratio (agent:hair) = 1:1 was applied on a hair tress of goat hair (manufactured by Beaulax Co., Ltd.). The mixture was left to stand for 30 minutes at 30°C, and then was rinsed with water at about 40°C, shampooed with a commercially available shampoo, and washed with water. A commercially available hair rinse was applied and then rinsed with water. The hair tress was dried with a towel. The color tone of the thus-treated and -obtained hair tress was measured based on the CIE color system (L*, a*, b*), using a colorimeter (color difference meter CR-400 manufactured by Konica Minolta Sensing, Inc.), and thus ΔE* was calculated by the following expression. In addition, the measurement was performed using 3 or more specimens, and the deviation between the average value and the respective measured values was within ± 1.2. The average value is presented in Table 3. A larger value of ΔE* means superior dyeing properties.

[Mathematical formula 1]

$$\Delta E^* = \sqrt{(L^*_2 - L^*_1)^2 + (a^*_2 - a^*_1)^2 + (b^*_2 - b^*_1)^2}$$

wherein $L^*_1$, $a^*_1$ and $b^*_1$ respectively represent the values of L*, a* and b* before dyeing; and $L^*_2$, $a^*_2$ and $b^*_2$ respectively represent the values of L*, a* and b* after dyeing.

[Table 1]

| First agent (component) | | Example (mass%) | | |
|---|---|---|---|---|
| | | 1 | 2 | 3 |
| (A) | Cationic anthraquinone direct dye (A-1) | 1.0 | 1.0 | 1.0 |
| (B) | Toluene-2,5-diamine (B-2) | 1.0 | 1.0 | 1.0 |
| | Resorcine | 1.0 | - | - |
| | Meta-aminophenol | - | 1.0 | - |
| | 5-Amino-ortho-cresol | 4.0 | - | 1.0 |
| | Propylene glycol | 4.0 | 4.0 | 4.0 |
| | Ascorbic acid | 0.4 | 0.4 | 0.4 |
| | Sodium sulfite | 0.4 | 0.4 | 0.4 |
| | EDTA-4Na | 0.1 | 0.1 | 0.1 |
| (C) | Steartrimonium chloride | 1.4 | 1.4 | 1.4 |
| | Ceteth-40 | 2.5 | 2.5 | 2.5 |
| | Cetearyl alcohol | 8.0 | 8.0 | 8.0 |

(continued)

| First agent (component) | | Example (mass%) | | |
|---|---|---|---|---|
| | | 1 | 2 | 3 |
| | Dimethicone*1 | 2.0 | 2.0 | 2.0 |
| | Amodimethicone*2 | 1.0 | 1.0 | 1.0 |
| | Aqueous ammonia (28 mass%) | 6.5 | 6.5 | 6.5 |
| | Fragrance | 0.5 | 0.5 | 0.5 |
| | Ethanolamine*3 | Appropriate amount | Appropriate amount | Appropriate amount |
| | Water | Balance | Balance | Balance |
| Total | | 100 | 100 | 100 |
| *1: SH 200C, Dow Corning Toray Co., Ltd.<br>*2: SM8704C, Dow Corning Toray Co., Ltd.<br>*3: Amount to adjust the pH to 10.7 | | | | |

[Table 2]

| Second agent A (component) | | (mass%) |
|---|---|---|
| | Cetanol | 2.0 |
| (C) | Sodium lauryl sulfate | 1.0 |
| | Aqueous hydrogen peroxide (35 mass%) | 17.0 |
| | Methylparaben | 0.1 |
| | Phosphoric acid | Appropriate amount*4 |
| | Water | Balance |
| Total | | 100 |
| *4: Amount to adjust the pH to 3.5 | | |

[Table 3]

| | Example | | |
|---|---|---|---|
| | 1 | 2 | 3 |
| pH (25°C) after mixing of first agent and second agent | 9.6 | 9.6 | 9.6 |
| $\Delta L^*(L^*_2-L^*_1)$ | 22.1 | 18.5 | 16.1 |
| $\Delta a^*(a^*_2-a^*_1)$ | 1.4 | 1.4 | 6.5 |
| $\Delta b^*(b^*_2-b^*_1)$ | 5.3 | 2.3 | -1.8 |
| $\Delta E^*$ | 22.8 | 18.7 | 17.5 |
| Color tone of dyed hair tress | Deep green brown color | Deep blue brown color | Deep violet brown color |

As described above, all of the hair dyes were able to dye the hair tresses of goat hair in deep colors. Furthermore, even in the case where hair dyeing was performed after the first agent of Example 2 was stored for 2 months at 50°C, the hair was dyed in the same color tone. It was not possible to distinguish by visual inspection between the color tone of the hair tress dyed using the first agent immediately after production, and the color tone of the hair tress

dyed using the first agent after storage for 2 months.

[Shampoo-fastness test]

An aqueous solution (10% by mass) of the test shampoo indicated in Table 4 was filled in a 100-mL glass bottle, and 3 specimens each of the dyed hair tresses obtained by dyeing with the hair dyes of Examples 1 to 3 were immersed in the bottle. Shampooing of the hair tresses was performed over 20 minutes while the bottle was shaken in a shaking agitator (40°C, 120 rpm), and thus shampoo-fastness was evaluated. The shampooing effect obtained under these conditions corresponds to regular daily shampooing for about one week.

[Table 4]

| Test shampoo (component) | (mass%) |
|---|---|
| Sodium laureth-2 sulfate | 55.0 |
| EDTA-4Na | 1.0 |
| Sodium benzoate solution (35 mass%) | 3.0 |
| Lauramide DEA | 13.0 |
| Sodium chloride | 4.5 |
| Phosphoric acid (75 mass%) *5 | Appropriate amount |
| Purified water | Balance |
| Total | 100 |
| *5: Amount to adjust the pH to 7 | |

The dyed hair tresses obtained by dyeing with the hair dyes of Examples 1 to 3 all underwent slight color thinning, but the respective hair tresses maintained the color tones of deep green brown color, deep blue brown color and deep violet brown color.

Examples 4 to 7

A first agent of a cream-type two-part type hair dye as indicated in Table 5, and a common second agent B indicated in Table 6 were prepared, and the first agent and the common second agent B were mixed at a mass ratio of first agent:common second agent B = 1:1, to obtain a hair dye composition. These hair dye compositions were applied, respectively in equal mass amounts, to about 1 g of a hair tress made of human hair mixed with 10% of grey hair, and were left to stand for 30 minutes. The hair dye compositions were washed with water, shampooed, washed and dried, and thus, dyed hair tresses having satisfactory dyeing properties and shampoo-fastness were obtained.

[Table 5]

| First agent (component) | | Example (mass%) | | | |
|---|---|---|---|---|---|
| | | 4 | 5 | 6 | 7 |
| (A) | Cationic anthraquinone direct dye (A-1) | 0.5 | 0.8 | 0.1 | 1.0 |
| (B) | Paraphenylenediamine (B-1) | - | 0.2 | - | - |
| | Toluene-2,5-diamine (B-2) | 0.4 | - | 0.4 | - |
| | 2-(2-Hydroxyethyl) paraphenylenediamine (B-3) | - | 0.2 | 0.4 | 0.3 |

(continued)

| First agent (component) | | Example (mass%) | | | |
|---|---|---|---|---|---|
| | | 4 | 5 | 6 | 7 |
| | Para-aminophenol | - | 0.1 | 0.3 | 0.1 |
| | Meta-aminophenol | - | 0.2 | 0.2 | - |
| | 5-Amino-ortho-cresol | 0.3 | - | 0.2 | 0.1 |
| (C) | Cocamide MEA | 4.5 | 4.5 | 4.5 | 4.5 |
| | Glyceryl stearate (SE) | 1.3 | 1.3 | 1.3 | 1.3 |
| | Ceteareth-30 | 4.0 | 4.0 | 4.0 | 4.0 |
| | Sodium lauryl sulfate | 1.0 | 1.0 | 1.0 | 1.0 |
| | Ammonia (28 mass%) | 6.0 | 6.0 | 6.0 | 6.0 |
| | Stearyl alcohol | 8.0 | 8.0 | 8.0 | 8.0 |
| | Oleic acid | 2.0 | 2.0 | 2.0 | 2.0 |
| | Propylene glycol | 1.5 | 1.5 | 1.5 | 1.5 |
| | PEG-9 dimethicone*6 | 1.5 | 1.5 | 1.5 | 1.5 |
| | Hydrolyzed keratin | 0.5 | 0.5 | 0.5 | 0.5 |
| | Panthenol | 0.8 | 0.8 | 0.8 | 0.8 |
| | EDTA-4Na | 0.5 | 0.5 | 0.5 | 0.5 |
| | Ammonium chloride | Appropriate amount*7 | Appropriate amount*7 | Appropriate amount*7 | Appropriate amount*7 |
| | Purified water | Balance | Balance | Balance | Balance |
| Total | | 100.0 | 100.0 | 100.0 | 100.0 |
| *6: KF-6005, Shin-Etsu Chemical Co., Ltd.<br>*7: Amount to adjust the pH to 10 | | | | | |

[Table 6]

| Second agent B (component) | | (mass%) |
|---|---|---|
| | Cetanol | 2.0 |
| (C) | Sodium lauryl sulfate | 1.0 |
| | Hydrogen peroxide (50 mass%) | 12.0 |
| | Methylparaben | 0.1 |
| | Phosphoric acid | Appropriate amount*8 |
| | Purified water | Balance |
| Total | | 100.0 |
| *8: Amount to adjust the pH to 3.5 | | |

Examples 8 to 11

A first agent of a two-part type hair dye as indicated in Table 7 was prepared, and the first agent and the common second agent B were mixed at a mass ratio of first agent:common second agent B = 1:1, and thus a hair dye composition was obtained. These hair dye compositions were applied, respectively in equal mass amounts, to about 1

g of a hair tress made of human hair mixed with 10% of grey hair, and were left to stand for 30 minutes. The hair dye compositions were washed with water, shampooed, washed and dried, and thus, dyed hair tresses having satisfactory dyeing properties and shampoo-fastness were obtained.

[Table 7]

| First agent (component) | | Example (mass%) | | | |
|---|---|---|---|---|---|
| | | 8 | 9 | 10 | 11 |
| (A) | Cationic anthraquinone direct dye (A-1) | 0.3 | 0.1 | 0.6 | 0.5 |
| | Direct dye X | - | - | 0.2 | - |
| | Direct dye Y | - | - | - | 0.4 |
| | Direct dye Z | - | 0.3 | - | - |
| (B) | Toluene-2,5-diamine sulfate (B-2) | 0.2 | 0.1 | - | - |
| | 2-(2-Hydroxyethyl)paraphenylenediamine (B-3) | - | 0.3 | - | 0.4 |
| | N,N-bis(2-hydroxyethyl)paraphenylenediamine (B-5) | - | - | 0.5 | 0.2 |
| | Para-aminophenol | - | 0.3 | 0.2 | - |
| | Meta-aminophenol | 0.2 | - | 0.2 | - |
| | 5-Amino-ortho-cresol | - | 0.3 | - | 0.4 |
| (C) | Behentrimonium chloride | 2.1 | 2.1 | 2.1 | 2.1 |
| | Mineral oil | 0.5 | 0.5 | 0.5 | 0.5 |
| | Propylene glycol | 7.0 | 7.0 | 7.0 | 7.0 |
| | Cetearyl alcohol | 7.0 | 7.0 | 7.0 | 7.0 |
| | Aqueous ammonia (28 mass%) | 6.5 | 6.5 | 6.5 | 6.5 |
| | Polyquaternium-10[*9] | 1.0 | - | - | 1.0 |
| | Amodimethicone[*10] | 1.5 | 1.5 | 1.5 | - |
| | Purified water | Balance | Balance | Balance | Balance |

(continued)

| First agent (component) | Example (mass%) | | | |
|---|---|---|---|---|
| | 8 | 9 | 10 | 11 |
| Total | 100.0 | 100.0 | 100.0 | 100.0 |

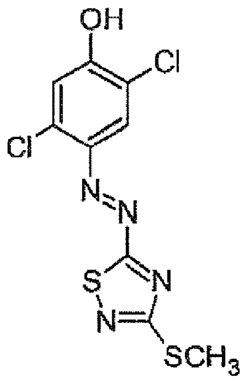

Direct dye X

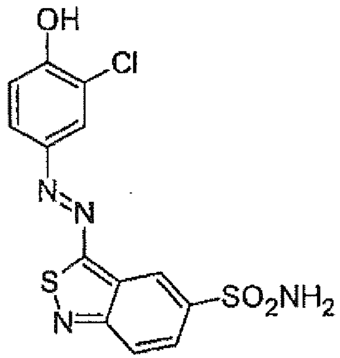

Direct dye Y

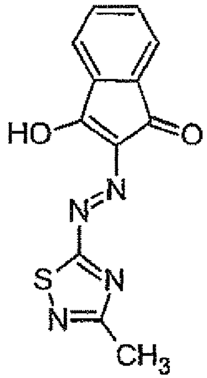

Direct dye Z

*9: Ucare Polymer JR-400, Amerchol Corp.

*10: SM8704C, Dow Corning Toray Co., Ltd.

Examples 12 to 15

A first agent of a two-part type hair dye as indicated in Table 8, and a booster liquid were prepared, and the first agent, the booster liquid, and the common second agent B were mixed at a mass ratio of first agent: booster liquid: common second agent = 1:0.1:1. Thus, a hair dye composition was obtained. These hair dye compositions were applied, respectively in equal mass amounts, to about 1 g of a hair tress made of human hair mixed with 10% of grey hair, and were left to stand for 30 minutes. The hair dye compositions were washed with water, shampooed, washed and dried, and thus, dyed hair tresses having satisfactory dyeing properties and shampoo-fastness were obtained.

[Table 8]

| Component | | | Example (mass%) | | | |
|---|---|---|---|---|---|---|
| | | | 12 | 13 | 14 | 15 |
| First agent | (A) | Cationic anthraquinone direct dye (A-1) | 0.3 | 0.2 | 0.1 | - |
| | | HC Red 3 | 1.0 | - | 0.3 | - |
| | (B) | Paraphenylenediamine (B-1) | 0.2 | - | 0.4 | - |
| | | Toluene-2,5-diamine sulfate (B-2) | - | 0.6 | - | 0.5 |
| | | Para-aminophenol | - | 0.2 | 0.2 | 0.1 |
| | | 5-Amino-ortho-cresol | - | 0.2 | 0.2 | 0.1 |
| | | Meta-aminophenol | 0.2 | - | 0.2 | - |
| | (C) | Behentrimonium chloride | 2.1 | 2.1 | 2.1 | 2.1 |
| | | Mineral oil | 0.5 | 0.5 | 0.5 | 0.5 |
| | | Propylene glycol | 7.0 | 7.0 | 7.0 | 7.0 |
| | | Cetearyl alcohol | 7.0 | 7.0 | 7.0 | 7.0 |
| | | Aqueous ammonia (28 mass%) | 6.5 | 6.5 | 6.5 | 6.5 |
| | | Polyquaternium-10*11 | 1.0 | - | 1.0 | - |
| | | Amodimethicone*12 | 1.5 | 1.5 | 1.5 | 1.5 |
| | | Purified water | Balance | Balance | Balance | Balance |
| | Total | | 100.0 | 100.0 | 100.0 | 100.0 |
| Booster liquid | Cationic anthraquinone direct dye (A-1) | | 1.0 | 2.5 | - | 2.0 |
| | Basic Red 51 | | - | 0.4 | 1.5 | - |
| | Aqueous ammonia (28 mass%) | | 2.0 | 2.0 | 2.0 | 2.0 |
| | PEG-8 | | 3.0 | 3.0 | 3.0 | 3.0 |
| | Purified water | | Balance | Balance | Balance | Balance |
| | Total | | 100.0 | 100.0 | 100.0 | 100.0 |
| *11: Ucare Polymer JR-400, Amerchol Corp.<br>*12: SM8704C, Dow Corning Toray Co., Ltd. | | | | | | |

**Claims**

**1.** A hair dye composition for utilizing a first agent containing an alkali agent and a second agent containing an oxidizing agent in mixture, the composition comprising the following component (A) and component (B):

Component (A): Cationic anthraquinone direct dye represented by formula (1)

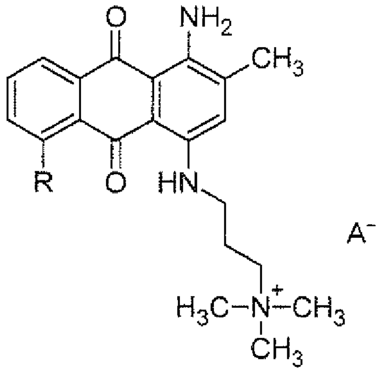

(1)

wherein R represents a hydrogen atom or an amino group; and $A^-$ represents an anion;
Component (B): A compound represented by formula (2) or a salt thereof

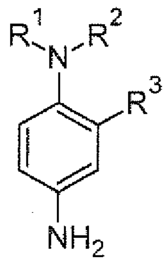

(2)

wherein $R^1$ represents a hydrogen atom, a methyl group or a hydroxyethyl group; $R^2$ represents a hydrogen atom, a methyl group, a phenyl group, a hydroxyethyl group, or a 3-(N-hydroxyethyl-N-(4-aminophenyl)amino)-2-hydroxypropyl group; and $R^3$ represents a hydrogen atom, a hydroxyl group, a methyl group, a hydroxyethyl group, or a hydroxyethoxy group.

**2.** The hair dye composition according to claim 1, further comprising a component (C) surfactant.

**3.** The hair dye composition according to claim 1 or 2, wherein the content of the component (A) is 0.01% to 5% by mass relative to the whole composition, and the content of the component (B) is 0.1% to 5% by mass relative to the whole composition.

**4.** A method of dyeing hair by applying the hair dye composition according to any one of claims 1 to 3 to the hair.

**5.** A method of dyeing hair by mixing the first agent and the second agent of the hair dye composition according to any one of claims 1 to 3 before use, subsequently applying the mixture to the hair, leaving the hair to stand, rinsing the hair dye composition, and drying the hair.

**6.** The method of dyeing hair according to claim 5, wherein after the hair dye is rinsed, the hair is washed using a shampoo containing an anionic surfactant and then washed with water.

**INTERNATIONAL SEARCH REPORT**

International application No.
PCT/JP2009/003955

A. CLASSIFICATION OF SUBJECT MATTER
*A61K8/41*(2006.01)i, *A61Q5/10*(2006.01)i

According to International Patent Classification (IPC) or to both national classification and IPC

B. FIELDS SEARCHED

Minimum documentation searched (classification system followed by classification symbols)
A61K8/41, A61Q5/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | EP 1820826 A1 (DyStar Textilfarben GmbH & Co. Deutschland KG., Kao Corp.), 22 August 2007 (22.08.2007), claims; examples 1, 2, A, B & WO 2007/090799 A2 & WO 2007/090800 A2 | 1-6 |
| Y | JP 04-230619 A (L'Oreal), 19 August 1992 (19.08.1992), claims (particularly, claim 13); paragraphs [0021] to [0024], [0030] to [0032]; examples & US 5226924 A & EP 0480829 A1 | 1-6 |
| Y | JP 2002-226337 A (Hoyu Co., Ltd.), 14 August 2002 (14.08.2002), claims; paragraphs [0012], [0013]; examples (Family: none) | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 November, 2009 (16.11.09) | 24 November, 2009 (24.11.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/003955

C (Continuation). DOCUMENTS CONSIDERED TO BE RELEVANT

| Category* | Citation of document, with indication, where appropriate, of the relevant passages | Relevant to claim No. |
|---|---|---|
| Y | JP 08-175939 A (L'Oreal), 09 July 1996 (09.07.1996), claims (particularly, claims 1, 7); paragraph [0028] & US 5928385 A & EP 0705598 A1 | 1-6 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1820826 A1
- JP 2002275040 A
- JP 2003107222 A
- JP 2003107223 A
- JP 2003113055 A
- JP 2003342139 A
- JP 2004107343 A
- JP 2004155746 A
- JP 2004262888 A
- JP 2006182653 A

### Non-patent literature cited in the description

- Hair and Hair Care. Marcel Dekker, 1997, 204-207